# EUROPÄISCHE PATENTSCHRIFT

(11) **EP 2 423 186 B1**
(45) Veröffentlichungstag und Bekanntmachung des Hinweises auf die Patenterteilung: **31.05.2017**
(21) Anmeldenummer: 11177949.2
(22) Anmeldetag: 18.08.2011
(51) Int. Cl.: C07C 267/00, C08K 5/29, C09J 161/12, C08J 5/06, D06M 15/41

(54) **NEUARTIGE HAFTVERMITTLER AUF BASIS VON CARBODIIMIDEN, HAFTVERMITTLERHALTIGE, WÄSSRIGE RESORCIN-FORMALDEHYD-LATEX-DISPERSIONEN, HAFTUNGSVERBESSERTE FASERN, VERFAHREN ZU DEREN HERSTELLUNG UND DEREN VERWENDUNG**
NEW ADHESION PROMOTER BSED ON CARBODIIMIDES, ADHESIVE, AQUEOUS RESORCINOL-FORMALDEHYDE-LATEX DISPERSIONS, FIBERS WITH IMPROVED ADHESION, PROCESS FOR THEIR PREPARATION AND USE THEREOF.
NOUVEAU AGENT ADHÉSIF BASÉ SUR DES CARBODIIMIDES, DISPERSIONS AQUEOUSES ET ADHÉSIVES RESORCINOL-FORMALDÉHYDE-LATEX, FIBRES AVEC ADHÉRENCE AMÉLIORÉE, LEUR PROCÉDÉ DE PRÉPARATION ET LEUR APPLICATION.

(30) Priorität: 30.08.2010 EP 10174548; 28.10.2010 EP 10189268; 22.11.2010 EP 10192089; 18.03.2011 EP 11158813
(43) Veröffentlichungstag der Anmeldung: 29.02.2012
(73) Patentinhaber: LANXESS Deutschland GmbH, 50569 Köln (DE)
(72) Erfinder: Laufer, Wilhelm, 67158 Ellerstadt (DE); Blaul, Anke, 64297 Darmstadt (DE); Eckert, Armin, 68794 Oberhausen-Rheinhausen (DE); Fruth, Andrea, 65189 Wiesbaden (DE); Cano Sierra, Ana Maria, 69117 Heidelberg (DE)

(56) Entgegenhaltungen:
- US-A- 3 661 623
- US-A- 4 159 363
- US-A- 4 263 221
- US-A- 4 477 619
- US-A- 5 498 747
- US-A1- 2006 094 851

## Beschreibung

Die vorliegenden Erfindung betrifft neuartige Haftvermittler auf Basis von Carbodiimiden, Haftvermittlerhaltige, wässrige Resorcin-Formaldehyd-Latex-Dispersionen, haftungsverbesserte Fasern, Verfahren zu deren Herstellung und deren Verwendung zur Haftungsverbesserung im Reifen. Carbodiimide werden häufig zur Behandlung von Reifencords eingesetzt, siehe US-A-3867181 und DE-A-1770495. Diese Behandlungen werden zur Verbesserung der Hydrolysestabilität von PET-Fasern durchgeführt. In DE-A-2326540 werden Polyisocyanate, die Polycarbodiimide beinhalten, beschrieben. Diese Verfahren basieren jedoch auf unerwünschten organischen Lösemitteln und sind unwirtschaftlich.

Resorcin-Formaldehyd-Latex-Dispersionen (RFL-Dip) haben sich insbesondere im Reifenbereich durchgesetzt, da diese die Haftung des Kunststoffgewebes (Cord) zum Gummi verbessern.

US3661623 offenbart Polyester-Fasern, die in einer wässrigen Resorcin-Formaldehyd-Latex-Dispersion enthaltend Styrol-Butadien-Vinylpyridin-Copolymer-Latex eingetaucht werden. Die Dispersion enthält ein Polycarbodiimid als Stabilisierungsmittel. Im Fall von Polyester als Cord-Material besteht jedoch der Nachteil, dass die haftungsvermittelnden Eigenschaften des RFL-Dips nicht ausreichend sind.

Es wurde daher versucht, durch Zugabe von dimeren Isocyanaten diesen Nachteil zu beseitigen, was allerdings an der geringen Performance und der geringere Lagerstabilität scheiterte.

Um die Haftung bei der Verwendung von Polyester-Cord zum Reifen/Gummi zu verbessern, werden dem RFL-Dip mit Caprolactamen geblockte Isocyanate zugesetzt (siehe US A 20080300347). Diese haben wiederum den Nachteil, dass im weiteren Prozeß toxische monomere Isocyanate und störendes Caprolactam abgespalten werden.

Des Weiteren ist aus EP-A 2159241 der Einsatz von mikroverkapseltem dimerem Diphenylmethan-4,4'-diisocyanat und Diphenylmethan-2,4-diisocyanat (MDI) zur Verbesserung der haftungsvermittelnden Eigenschaften bekannt. Die hier beschriebenen Substanzen haben jedoch die Nachteile, dass diese teuer und kommerziell nicht verfügbar sind und ebenfalls toxische monomere Diisocyanate abspalten können.

Aufgabe der vorliegenden Erfindung war daher, Haftvermittler bereitzustellen, die insbesondere in Resorcin-Formaldehyd-Latex-Dispersionen zur Verbesserung der Haftung einsetzbar sind und die Nachteile des Standes der Technik nicht aufweisen.

Überraschenderweise wurde nun gefunden, dass neuartige Haftvermittler, enthaltend carbodiimidisierte Isocyanate und/oder deren Umsetzungsprodukte mit Oberflächendesaktivierungsmitteln, wie Aminen, eine exzellente Haftung gewährleisten. Diese haben den Vorteil, dass diese bei der Verarbeitung im weiteren Prozeß, keine giftige monomere Isocyanate abspalten und durch einfache Produktionsverfahren hergestellt werden können.

Gegenstand der vorliegenden Erfindung sind daher Haftvermittler, enthaltend mindestens ein Carbodiimid auf Basis von Verbindungen der Formel (I)

R'-(-N=C=N-R-)ₘ-R" (I),

in der
m einer ganzen Zahl von 1 bis 500, bevorzugt von 1 bis 20 entspricht,
R = C₁-C₁₈-Alkylen, C₅-C₁₈-Cycloalkylen-, Arylen und/oder C₇-C₁₈-Aralkylen,
R' = R-NCO, R-NHCONHR¹, R-NHCONR¹R² oder R-NHCOOR³ und
R"= -NCO, -NHCONHR¹, -NHCONR¹R² oder -NHCOOR³ ist,
wobei in R' unabhängig voneinander R¹ und R² gleich oder verschieden sind und einen C₁-C₆-Alkyl-, C₆-C₁₀-Cycloalkyl- oder C₇-C₁₈-Aralkylrest darstellen und R³ eine der Bedeutungen von R¹ hat oder einen Polyester- oder einen Polyamidrest oder -(CH₂)₁-(O-(CH₂)ₖ-O)_{g}-R⁴, -C₆H₄(OH) oder - C₆H₃(OH)-((CH₂)ₕ-C₆H₄(OH))_{y},
mit 1 = 1-3, k= 1-3, g = 0-12 , h = 1-2 und y = 1-50
bedeutet und
R⁴ = H oder C₁-C₄-Alkyl,
welche durch Umsetzung mit mindestens einem Amin oberflächendesaktiviert wurden.

Ebenfalls einsetzbar sind auch Gemische von Carbodiiimiden der Formel (I), inklusive der entsprechenden Oligomere und/oder Polymere.

In einer besonders bevorzugten Ausführungsform der Erfindung entsprechen die Carbodiimide den Formeln (II) bis (IV) 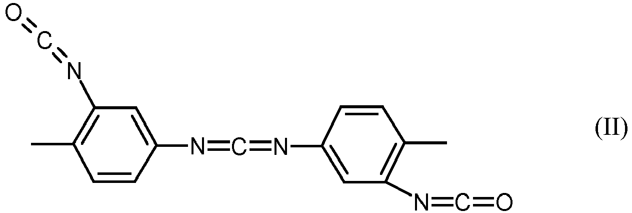 und/oder 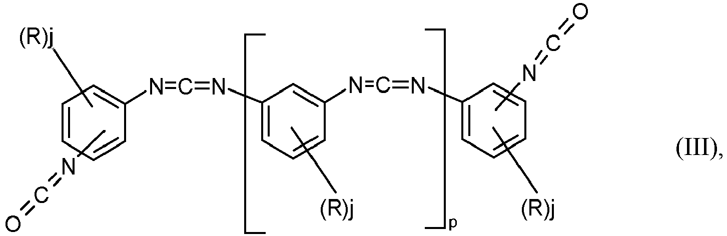 mit R = C₁-C₁₈-Alkylen, C₅-C₁₈-Cycloalkylen-, Arylen und/oder C₇-C₁₈-Aralkylen,
j innerhalb des Moleküls gleich oder verschieden ist und 1 bis 5 bedeutet und
p = 0 bis 500,
und/oder sterisch gehinderte Carbodiimide der Formel (IV) 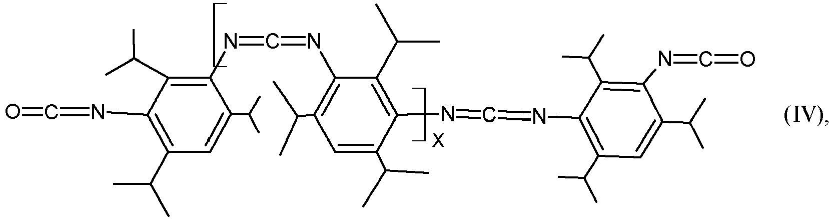 mit x = 1 bis 500, bevorzugt 1 - 50,
welche durch Umsetzung mit mindestens einem Amin oberflächendesaktiviert wurden.

Herstellungsbedingt fallen die Carbodiimide häufig im Gemischen aus monomeren, oligomeren und/oder polymeren Carbodiimiden an. Diese Gemische sind vom Gegenstand der Erfindung mitumfasst.

Ebenfalls einsetzbar sind Verbindungen der Formeln (I) bis (IV), die geblockt sind z.B. mit Lactamen, besonders bevorzugt Caprolactam, Phenolen, Novolacken, Resorcin, Oxim und/oder Epoxiden.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die Verbindungen nach Formel (I) bis (IV) vor der Oberflächendesaktivierung sind kommerziell erhältlich, z.B. bei der Firma Rhein Chemie Rheinau GmbH oder lassen sich nach den dem Fachmann geläufigen Verfahren herstellen, wie z.B. beschrieben in DE-A-11 30 594 oder US 2 840 589 oder durch die Kondensation von Diisocyanaten unter Abspaltung von Kohlendioxid bei erhöhten Temperaturen, z.B. bei 40 °C bis 200 °C, in Gegenwart von Katalysatoren. Als Katalysatoren haben sich z.B. starke Basen oder Phosphorverbindungen bewährt. Vorzugsweise werden Phospholenoxide, Phospholidine oder Phospholinoxide sowie die entsprechenden Sulfide verwendet. Ferner können als Katalysatoren tertiäre Amine, basisch reagierende Metallverbindungen, Carbonsäuremetallsalze und nicht basische Organometallverbindungen verwendet werden.

Zur Herstellung der eingesetzten Carbodiimide und/oder Polycarbodiimide eignen sich alle Diisocyanate, wobei im Rahmen der vorliegenden Erfindung bevorzugt Carbodiimide und/oder Polycarbodiimide verwendet werden, die auf C₁- bis C₄-Alkyl substituierten aromatischen Isocyanaten aufbauen, wie z.B. 2,4-Toluylendiisocyanat, 2,6-Toluylendiisocyanat, ein Gemisch aus 2,4-Toluylendiisocyanat und 2,6-Toluylendiisocyanat, Hexamethylendiisocyanat, Cyclohexan-1,4-diisocyanat, Xylylendiisocyanat, Isophorondiisocyanat, 2,6-Diisopropylphenylisocyanat, 2,4,6-Triisopropylphenyl-1,3-diisocyanat, 2,4,6-Triethylphenyl-1,3-diisocyanat, 2,4,6-Trimethylphenyl-1,3-diisocyanat, 2,4'-Diisocyanatodiphenylmethan, 3,3',5,5'-Tetraisopropyl-4,4'-diisocyanato-diphenylmethan, 3,3',5,5'-Tetraethyl-4,4'-di-isocyanatodiphenylmethan, Tetramethylxyloldiisocyanat, 1,5-Naphthalindiisocyanat, 4,4'-Diphenylmethandiisocyanat, 2,4'-Diphenyl-methandiisocyanat, 2,2'-Diphenylmethandiisocyanat, 4,4'-Diphenyldimethyl-methandiisocyanat, 1,3-Phenylendiisocyanat, 1,4-Phenylendiisocyanat, Dicyclohexylmethan-4,4'-diisocyanat, Dicyclohexylmethan-2,4'-diisocyanat, Dicyclohexylmethan-2,2'-diisocyanat, Methylcyclohexandiisocyanat, Tetramethylxylylendiisocyanat, 2,6-Diisopropylphenylen-isocyanat und 1,3,5-Triisopropylbenzol-2,4-diisocyanat oder deren Gemische, oder auf substituierten Aralkylen, wie 1,3-Bis-(1-methyl-1-isocyanato-ethyl)-benzol, basieren. Besonders bevorzugt ist es, wenn die Carbodiimide und/oder Polycarbodiimide auf 2,4-Toluylendiisocyanat und 2,6-Toluylendiisocyanat oder einem Gemisch aus 2,4-Toluylendiisocyanat und 2,6-Toluylendiisocyanat basieren.

In einer weiteren Ausführungsform der vorliegenden Erfindung ist es auch möglich, eine Mischung verschiedener Carbodiimide einzusetzen.

Die eingesetzten festen Carbodiimide weisen besonders bevorzugt eine Teilchengröße von < 50µm auf.

In einer Ausführungsform der Erfindung können die erfindungsgemäßen Haftvermittler dabei als wässrige Lösung und/oder wässrige Dispersion vorliegen und noch weitere Zusatzstoffe enthalten, wie z.B. rheologische Hilfsmittel (Antiabsetzmittel), wie z.B. Borchi®Gel ALA (OMG Borchers GmbH) oder Kelzan® S, erhältlich bei der Firma Monsanto, oder auch Tragacanth, erhältlich bei der Firma R.T. Vanderbilt, Stabilisatoren, Emulgatoren, Netz- und/oder Dispergiermittel, wie z.B. Tamol® NN 9104 der Firma BASF AG oder Aerosol® OT45 der Firma Cytec Surface Specialities GmbH, Dispersogen® HR der Firma Clariant International Ltd.

Die Anteile an Carbodiimiden in der wässrigen Dispersion betragen vorzugsweise 1-80%, besonders bevorzugt 40 - 60%.

Die Carbodiimide in dem erfindungsgemäßen Haftvermittler sind dabei gemäß den Formeln (I) bis (IV) durch Umsetzung mit mindestens einem Amin oberflächendesaktiviert.

Für die Oberflächendesaktivierung (Mikroverkapselung) sind als Amin alle aminofunktionellen Verbindungen einsetzbar. Bevorzugt handelt es sich dabei um multifunktionelle primäre und sekundäre, besonders bevorzugt multifunktionelle aliphatische Amine. Erfindungsgemäß geeignete Amine sind insbesondere ausgewählt aus der Gruppe von cyclischen und aliphatischen, geradkettigen oder verzweigten (C₂-C₁₄)-Alkylaminen, -diaminen und -polyaminen, insbesondere (C₂-C₁₀)-Alkylaminen, -diaminen und -polyaminen, vorzugsweise (C₂-C₆)-Alkylaminen, -diaminen und -polyaminen, wobei die Alkylkette zumindest teilweise oder aber vollständig durch Heteroatome, insbesondere Sauerstoff oder Schwefel, unterbrochen sein kann und/oder wobei die Alkylkette weitere Substituenten, wie beispielsweise Hydroxylgruppen, Carboxylgruppen, Halogen oder dergleichen enthalten kann.

Als Beispiele für erfindungsgemäß geeignete Amine lassen sich die folgenden Verbindungen nennen: 2-Methyl-Pentamethylen-1,5-diamin und dessen Isomere und Homologe wie z. B. 1,6-Hexamethylendiamin; Di-sek.-Butylamin; Ethylendiamin; 1,3-Propylendiamin; Diethylen-triamin; Triethylentetramin; 3,3'-Dimethyl-4,4'-diaminodicyclohexylmethan; Methylnonandiamin; Isophorondiamin; 4,4'-Diaminodicyclohexylmethan; Alkanolamine und -diamine wie Ethanolamin und Diethanolamin und/oder Amidoamine. Hierunter ganz besonders bevorzugt sind 2-Pentamethylen-1,5-diamin und dessen Isomere und Homologe wie z. B. 1,6-Hexamethylendiamin.

Besonders bevorzugt handelt es sich dabei um multifunktionelle primäre und sekundäre, besonders bevorzugt multifunktionelle aliphatische Amine, wie z.B. Jeffamine® T 403 der Firma Huntsman, Diisopropanolamin der Firma BASF AG oder Amidoamine, wie Versamid® 140 der Firma Cognis oder Euretek 505 der Firma Witco. Dabei handelt es sich insbesondere um Verbindungen mit hydrophilen Gruppen, wie insbesondere Aminogruppen oder Hydroxylgruppen, die mit den freien Isocyanatgruppen des festen Diisocyanats reagieren können und somit eine Oberflächenhülle auf den Isocyanaten ausbilden, welche die Isocyanate zunächst desaktiviert, so z.B. Amine, Diamine und Polyamine.

In einer bevorzugten Ausführungsform der Erfindung wird als Oberflächendesaktivierungsmittel ein niedermolekulares Mono-, Di- oder Polyamin mit einer oder mehreren primären und/oder sekundären Aminogruppe(n) verwendet, und zwar in solchen Mengen, dass der Desaktivierungsgrad (DG), berechnet als Äquivalentverhältnis von Aminogruppen des Oberflächendesaktivierungsmittels zu den Isocyanat- und/oder Carbodiimidgruppen des zu desaktivierenden Carbodiimides (n NH₂/n NCO), zwischen 0,2 und 8 Äquivalent-% liegt.

Insbesondere kann das Oberflächendesaktivierungsmittel ein Molekulargewicht bis MG 600 g/mol aufweisen.

Dabei sind Konzentrationen an Oberflächendesaktivierungsmittel (Amin), bezogen auf die Menge an Haftvermittler, von 1 bis 10 Gew.% bevorzugt, insbesondere bevorzugt 2 bis 5 Gew.%.

Die Oberflächendesaktivierung erfolgt vorzugsweise durch Zugabe des Amins zu einer wässrigen Dispersion des Carbodiimides, die gegebenenfalls noch Dispergiermittel und Antiabsatzmittel enthält, unter Rühren und/oder Vermahlen. Es ist aber auch möglich, die Oberflächendesaktivierung durch Zugabe des Amins in eine organische Dispersion, z.B. in Alkohol, Toluol, etc., des Carbodiimides durchzuführen.

Für den Rühr-/Vermahlprozess können handelsübliche Maschinen eingesetzt werden, wie z.B. Perlmühle, Dissolver und/oder Blattrührer.

Die Desaktivierung der Carbodiimide erfolgt in an sich bekannter Weise, siehe insbesondere EP 0 205 970 A und US-A-4 888 124, deren Inhalt hiermit in vollem Umfang durch Bezugnahme eingeschlossen ist, z.B. durch:
a) Dispergieren des pulverförmigen festen Carbodiimides in einer Lösung des Amins oder
b) Zugabe des Amins oder einer Lösung des Amins zu einer Dispersion der festen feinteiligen Carbodiimides.

Die Oberflächendesaktivierung kann dabei in Wasser und/oder in organischen Lösemitteln erfolgen.

Ein weiterer Gegenstand der vorliegenden Erfindung sind Resorcin-Formaldehyd-Latex-Dispersionen enthaltend die erfindungsgemäßen Haftvermittler der Formeln (I) bis (IV).

Bei der Resorcin-Formaldehyd-Latex-Dispersion im Sinne der Erfindung handelt es sich um eine Dispersion der Einzelkomponenten Resorcin und Formaldehyd und/oder Formaldehyd zusammen mit einem Vorkondensat aus Resorcin und Formaldehyd (z.B. Rhenosin® T der Firma Rhein Chemie Rheinau GmbH und Penacolite® 50 erhältlich bei der Firma Indspec Chemical Corp.) und einer oder mehreren der nachstehend genannten Latex-Dispersionen.

Als Latex-Dispersion im Sinne der Erfindung kommen alle im Stand der Technik bekannten Latices in Frage, wie z.B. XSBR-Latex (carboxylierte Styrol-Butadien-Copolymere), HS-SBR-Latex (Styrol-Butadien-Copolymere), Nitril-Butadien-Copolymere (NBR-Latex), CR-Latex (Polychloropren), PSBR-Latex (Pyridin-Styrol-Butadien-Copolymere) und/oder Acrylat-Latex (Reinacrylat- und Styrol-Acrylat-Copolymere) und/oder Styrol-Butadien-Vinylpyridin-Copolymer-Latices, wobei Styrol-Butadien-Vinylpyridin-Copolymer-Latices (z.B. Pliocord VP 106, erhältlich bei der Firma Eliochem) bevorzugt sind. Dabei handelt es sich um handelsübliche Substanzen, die z.B. erhältlich sind bei der Polymer Latex GmbH oder der Firma Eliokem.

Die Resorcin-Formaldehyd-Latex-Dispersion wird dabei vorzugsweise durch Einrühren einer basischen wässrigen Mischung aus Resorcin und Formaldehyd oder vorzugsweise einer basischen wässrigen Mischung aus Formaldehyd und dem Vorkondensat aus Resorcin und Formaldehyd in einer basischen wässrigen Latexmischung erhalten.

Das Verhältnis von Resorcin zu Formaldehyd beträgt vorzugsweise 1:1 bis 2,5: 1.

Das Verhältnis von Latex, bezogen auf dessen Festkörperanteil, zu dem Kondensat aus Resorcin und Formaldehyd liegt vorzugsweise bei 10:1 bis 4: 1, besonders bevorzugt 6:1.

Bei den eingesetzten wässrigen basischen Lösungen handelt es sich vorzugsweise um wässrige Natrium-Hydroxid und/oder Ammonium-Hydroxid-Lösungen. Dabei sind pH-Werte von 10 - 11 bevorzugt.

Die Carbodiimide und/oder oberflächendesaktivierten Carbodiimide werden dabei vorzugsweise in Mengen von 0,5 bis 10 %, besonders bevorzugt 5 - 8 %, bezogen auf den Feststoffanteil in der Resorcin-Formaldehyd-Latex-Dispersion, eingesetzt.

Gegenstand der vorliegenden Erfindung ist zudem ein Verfahren zur Herstellung der erfindungsgemäßen Resorcin-Formaldehyd-Latex-Dispersionen, wonach mindestens einer der erfindungsgemäßen Haftvermittler, enthaltend mindestens eine Verbindung der Formel (I) bis (IV) in die Resorcin-Formaldehyd-Latex-Dispersion eingerührt wird.

Gegenstand der vorliegenden Erfindung ist zudem ein weiteres Verfahren zur Herstellung der erfindungsgemäßen Resorcin-Formaldehyd-Latex-Dispersionen, enthaltend mindestens einen der erfindungsgemäßen oberflächendesaktivierten Haftvermittler, wonach zur Oberflächendesaktivierung mindestens ein Carbodiimid nach den Formeln (I) bis (IV) mit mindestens einem Amin entweder
a) durch Dispergieren mindestens eines pulverförmigen Carbodiimids nach den Formeln (I) bis (IV) in einer Lösung mindestens eines Amins oder
b) durch Zugabe mindestens eines Amins oder einer Lösung mindestens eines Amins zu einer Dispersion mindestens eines der Carbodiimide nach den Formeln (I) bis (IV) hergestellt wird, und anschließend

in die Resorcin-Formaldehyd-Latex-Dispersion eingerührt oder die Resorcin-Formaldehyd-Latex-Dispersion in diese Lösungen aus a) oder b) eingerührt wird.

Das Einrühren der erfindungsgemäßen Haftvermittler, enthaltend die Carbodiimide und/oder oberflächendesaktivierten Carbodiimide, in die Resorcin-Formaldehyd-Latex-Dispersion oder der Resorcin-Formaldehyd-Latex-Dispersion in den erfindungsgemäßen Haftvermittler mit den Carbodiimiden und/oder mit den desaktivierten Carbodiimiden erfolgt dabei mit handelsüblichen Mischaggregaten, wie z.B. Rührkesseln und Dispergatoren.

Gegenstand der vorliegenden Erfindung sind zudem Haftmittelformulierungen, enthaltend mindestens eine erfindungsgemäße wässrige Resorcin-Formaldehyd-Latex-Dispersion und zusätzlich mindestens ein Aktivierungsmittel.

Aktivierungsmittel im Sinne der Erfindung sind z.B. Epoxide, wie Glycidylether GE 500 der Firma Raschig, Bisphenol A Epoxynovolac der Firma Editya Birla Chemical etc..

Zur Herstellung der Haftmittelformulierungen werden dabei vorzugsweise die erfindungsgemäßen Haftvermittler, enthaltend die Carbodiimide der Formeln (I) bis (IV) oder die durch Umsetzung mit mindestens einem Amin oberflächendesaktivierten Carbodiimide, in die Resorcin-Formaldehyd-Latex-Dispersion eingerührt und danach das Aktivierungsmittel zugegeben, wobei eine andere Dosierreihenfolge nicht ausgeschlossen ist.

Gegenstand der vorliegenden Erfindung ist zudem Verfahren zur Verbesserung der Haftung von Verstärkungsfasern auf vernetztem Kautschuk oder Elastomeren, wonach die Verstärkungsfasern (Fasern, Cord) in die erfindungsgemäße Haftmittelformulierung eingebracht und anschließend getrocknet werden oder

die Verstärkungsfasern (Fasern, Cord) in ein oder mehreren Schritten mit einem oder mehreren der Bestandteilen der erfindungsgemäßen Haftmittelformulierung behandelt werden.

Insbesondere bei der letztgenannten Behandlung in mehreren Schritten mit zunächst einem oder mehreren Bestandteilen der erfindungsgemäßen Haftmittelformulierung, kann die Faser zwischenzeitlich auch getrocknet werden.

Sofern das vorgenannte erfindungsgemäße Verfahren in mehreren Schritten mit einem oder mehreren Bestandteilen der erfindungsgemäßen Haftmittelformulierung durchgeführt wird, sind beispielsweise folgende Ausführungsformen möglich:
So kann beispielsweise die Verstärkungsfaser zunächst in mindestens ein Epoxid eingebracht, gegebenenfalls getrocknet, und anschließend in die erfindungsgemäße Resorcin-Formaldehyd-Latex-Dispersion eingebracht werden, die mindestens einen erfindungsgemäßen Haftvermittler enthält, d.h. mindestens ein Carbodiimid der Formeln (I) bis (IV) nach Umsetzung mit einem Amin oder
die Verstärkungsfaser wird zunächst in eine Dispersion aus mindestens einem Epoxid und mindestens einem erfindungsgemäßen Haftvermittler, der mindestens ein Carbodiimid der Formeln (I) bis (IV) nach Umsetzung mit einem Amin enthält, eingebracht, gegebenenfalls getrocknet, und anschließend in eine Latex-Dispersion, die auch Resorcin und Formaldehyd oder Formaldehyd und ein Resorcin-Formaldehyd-Vorkondensat enthält, eingebracht.

Bei dem vernetzten Kautschuk oder Elastomeren handelt es sich dabei vorzugsweise um (SBR)-Styrol-Butadien-Kautschuk, (BR-) Butadien-Kautschuk, (NR-) Naturkautschuk, (IR-) synthetischer Naturkautschuk, Polyurethan-Elastomere oder Mischungen hieraus.

In den vorgenannten Fällen können sowohl voraktivierte (vorbehandelte), als auch nicht voraktivierte Verstärkungsfasern eingesetzt werden.

Bei den voraktivierten (vorbehandelten) Verstärkungsfasern handelt es sich um z.B. polyester- oder aramidbasierte Fasern, die während ihrer Herstellung (Spinnen) mit einer Schlichte behandelt wurden. Kommerziell erhältliche Produkte sind z.B. bei der Firma KoSa unter KoSa Type 793 und KoSa Type 748. Die Schlichten enthalten in vielen Fällen Epoxide.

Bei den nicht-vorbehandelten Verstärkungsfasern handelt es sich um z.B. polyester- oder aramidbasierte Fasern. Kommerziell erhältliche Produkte sind z.B. KoSa Type 792.

Ebenfalls mitumfaßt von dieser Erfindung ist auch ein Verfahren zur Verbesserung der Haftung von Verstärkungsfasern auf vernetztem Kautschuk oder Elastomeren, wonach voraktivierte (vorbehandelte) Verstärkungsfasern in die erfindungsgemäße wässrige Resorcin-Formaldehyd-Latex-Dispersion eingebracht und anschließend getrocknet werden.

Unter dem Begriff Fasern werden im Sinne der Erfindung neben Fasern auch Garne, Cords, als auch Verstärkungsgewebe verstanden, auf Basis von z.B. Polyester oder Aramid, wie u.a. Polyethylenterephthalat-basierte Fasern.

Gegenstand der vorliegenden Erfindung sind zudem haftungsverbesserte Fasern, erhältlich durch Inkontaktbringen der mit Aktivierungsmittel vorbehandelten Fasern mit mindestens einer erfindungsgemäßen wässrigen Resorcin-Formaldehyd-Latex-Dispersion oder durch Inkontaktbringen einer nicht-vorbehandelten Faser mit mindestens einer erfindungsgemäßen Haftmittelformulierung, und einer anschließenden Trocknung (Fixierung) bei Temperaturen von 180 - 260 °C.

Gegenstand der vorliegenden Erfindung ist zudem die Verwendung der erfindungsgemäßen Resorcin-Formaldehyd-Latex-Dispersion gegebenenfalls in Anwesenheit von Aktivierungsmitteln zur Verbesserung der Haftfestigkeit zwischen Verstärkungsfasern und Elastomeren in Reifen, Antriebsriemen, Transportbändern und/oder Schläuchen, ebenso wie die

Verwendung des erfindungsgemäßen Haftvermittlers gegebenenfalls in Anwesenheit von Aktivierungsmitteln zur Verbesserung der Haftfestigkeit zwischen Verstärkungsfasern und Elastomeren in Reifen, Antriebsriemen, Transportbändern und/oder Schläuchen.

Die nachfolgenden Beispiele dienen der Erläuterung der Erfindung, ohne dabei limitierend zu wirken.

### Ausführungsbeispiele:

### Verwendete Chemikalien:

TDI-Carbodiimid, ein Carbodiimid gemäß Formel (II), welches gegebenenfalls Anteile an Oligomer enthalten kann,
Addolink® CBM, Caprolactamblockiertes MDI (4,4-Diphenylmethandiisocyanat), erhältlich bei der Fa. Rhein Chemie Rheinau GmbH,
Tamol®NN 9104, Netz/Dispergiermittel, erhältlich bei der Firma BASF AG,
Borchi Gel ® L 75, Antiabsetzmittel, erhältlich bei der Firma OMG Borchers GmbH,
Aerosol® OT 75, Netz/Dispergiermittel, erhältlich bei der Firma Cytec Surface Specialties GmbH,
Kelzan® S, Antiabsetzmittel, erhältlich bei der Firma Monsanto,
Jeffamin® T 403, Polyetheramin , erhältlich bei der Firma Huntsman International LLC,
Penacolite® 50, ein Resorcin-Formaldehyd-Vorkondensat, erhältlich bei der Firma Indspec Chemical Corp sowie

Pliocord® VP 106, ein Styrol-Butadien-Vinylpyridin-Copolymer-Latex mit 41 % Feststoffanteil, erhältlich bei der Firma Eliokem.

Tabelle 1 fasst die Einsatzmengen zur Herstellung einer wässrigen Dispersion zusammen:

**Tabelle 1:**

| **Material** | **Bsp**. **1** | **Bsp. 2** |
|---|---|---|
| TDI-Carbodiimid | 80 | |
| Addolink ® CBM | | 100 |
| Tamol® NN 9104 | 4,4 | |
| Aerosol® OT 75 | | 2,4 |
| Wasser | 85,6 | 91 |
| Borchi Gel ® L 75 | 0,5 | |
| Jeffamin® T 403 | 2,0 | |
| Kelzan® S, 3% in Wasser | | 9,0 |

| | | |
|---|---|---|
| Die Einsatzmengen sind in Gew. Teilen angegeben. | | |

Die wässrigen Dispersionen wurden dabei wie folgt hergestellt:
Wasser und Netz/Dispergiermittel (Aerosol® OT 75 bzw. Tamol®NN 9104) wurden zusammengegeben und gelöst/gemischt. Anschließend wurde TDI-Carbodiimid, bzw. Addolink® CBM zugesetzt und im Dissolver homogenisiert. In Beispiel 1 wurde anschließend wurde Jeffamin T 403 zugegeben und unter Vermeidung von Scherkräften eingemischt. Danach wurde Borchi Gel®L 75 bzw. die frisch hergestellte Kelzan® S- Präparation eingemischt und homogenisiert.

Tabelle 2 gibt die Zusammensetzungen von Haftmittelformulierungen zur Behandlung von voraktivierten Polyesterfasern wieder:

**Tabelle 2:**

| **Material** | **Bsp. 3 (erf)** | **Bsp. 4 (VV)** | **Bsp. 5 (VV)** |
|---|---|---|---|
| wässrige TDI-Carbodimid-Dispersion gemäß Bsp. 1 | 28 | | |
| wässrige Addolink® CBM-Dispersion gemäß Bsp. 2 | | 28 | |
| Wasser | 367,3 | 367,3 | 395,3 |
| Natronlauge (10 % -ig) | 6 | 6 | 6 |
| Penacolite® 50 | 42,4 | 42,4 | 42,4 |
| Formaldehyd (37 %-ig) | 20,5 | 20,5 | 20,5 |
| Pliocord VP 106 | 411 | 411 | 411 |
| Ammoniak (25 %-ig) | 24,7 | 24,7 | 24,7 |

| | | | |
|---|---|---|---|
| VV= Vergleichsbeispiel, erf= erfindungsgemäß, die Einsatzmengen sind in Gew. Teilen angegeben. | | | |

Die Vortrocknung der behandelten Fasern erfolgte bei ca. 135 °C für ca. 60 s, die Fixierung bei 230 °C für 120 s.

Vulkanisierung und Haftungsprüfung wurden nach ASTM D 4393 durchgeführt. Als Testelastomermischung kam Dunlop SP 5320, erhältlich bei der Firma Dunlop, und ein aktiviertes PET-Garn zur Anwendung. Die Ergebnisse der Haftprüfung werden in Tabelle 3 zusammengefasst:

**Tabelle 3:**

| **Test** | **Einheit** | **Beispiele** | | |
|---|---|---|---|---|
| | | **TDI-Carbodiimid (Bsp. 3)** | **Addolink CBM (Bsp. 4)** | **ohne Haftvermittler (Bsp. 5)** |
| Streifen-Test (Bedeckungsgrad) | % | < 10 | < 10 | < 10 |
| Streifen-Test (Haftung) | N/2,5cm | 250 - 300 | 250 - 300 | 200-250 |
| T-Test* | N | 121 | 122 | 115 |
| H-Test* | N | 121 | 117 | 110 |

| | | | | |
|---|---|---|---|---|
| * Durchschnittwert aus 10 Messungen | | | | |

Aus den Versuchen ist klar ersichtlich, dass die erfindungsgemäßen Haftvermittler eine extrem gut Haftung zeigen und dabei im Vergleich zum Stand der Technik deutlich leichter herstellbar und damit wirtschaftlicher sind und zudem bei der Trocknung im Rahmen der Thermofixierung keine giftigen momeren Isocyanate abspalten. Damit weisen die erfindungsgemäßen Haftvermittler ökologisch und arbeitstechnisch gegenüber den im Stand der Technik bekannten Verbindungen deutliche Vorteile auf.

## Patentansprüche

1. Haftvermittler enthaltend mindestens ein Carbodiimid auf Basis von Verbindungen der Formel (I)
R'-(-N=C=N-R-)ₘ-R" (I),
in der
m einer ganzen Zahl von 1 bis 500 entspricht,
R = C₁-C₁₈-Alkylen, C₅-C₁₈-Cycloalkylen, Arylen und/oder C₇-C₁₈-Aralkylen,
R' = R-NCO, R-NHCONHR¹, R-NHCONR¹R² oder R-NHCOOR³ und
R"= -NCO, -NHCONHR¹, -NHCONR¹R² oder -NHCOOR³ ist,
wobei in R' unabhängig voneinander R¹ und R² gleich oder verschieden sind und einen C₁-C₆-Alkyl-, C₆-C₁₀-Cycloalkyl- oder C₇-C₁₈-Aralkylrest darstellen und R³ eine der Bedeutungen von R¹ hat oder einen Polyester- oder einen Polyamidrest oder -(CH₂)₁-(O-(CH₂)ₖ-O)_{g}-R⁴, -C₆H₄(OH) oder -C₆H₃(OH)-((CH₂)ₕ-C₆H₄(OH))_{y}
mit l = 1-3, k = 1-3, g = 0-12, h = 1-2 und y = 1-50 bedeutet,
und
R⁴ = H oder C₁-C₄-Alkyl ist,
welche durch Umsetzung mit mindestens einem Amin oberflächendesaktiviert wurden.

2. Haftvermittler nach Anspruch 1, **dadurch gekennzeichnet, dass** es sich bei dem Carbodiimid um Verbindungen der Formeln II, III und IV 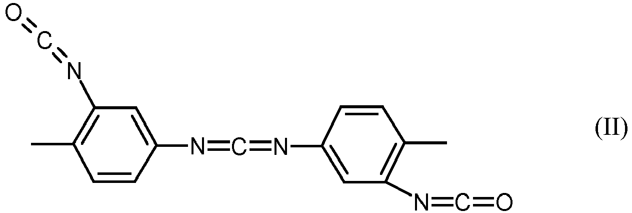 und/oder 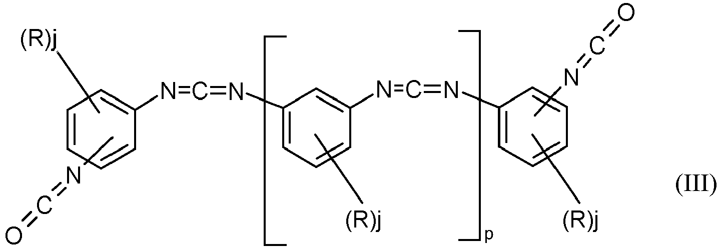 mit R = C₁ - C₁₈-Alkylen, C₅-C₁₈-Cycloalkylen, Arylen und/oder C₇-C₁₈-Aralkylen und j innerhalb des Moleküls gleich oder verschieden ist und 1 bis 5 bedeutet und p = 0 bis 500 ist,
und/oder 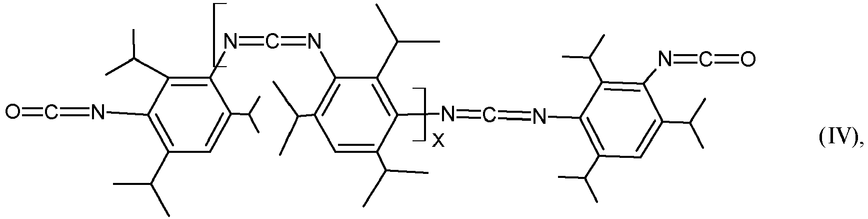 mit x = 1 bis 500 handelt,
welche durch Umsetzung mit mindestens einem Amin oberflächendesaktiviert wurden.

3. Haftvermittler nach Anspruch 1 oder 2, **dadurch gekennzeichnet, dass** es sich bei den Aminen um 1,6-Hexamethylendiamin; Di-sek.-Butylamin; Ethylendiamin; 1,3-Propylendiamin; Diethylen-triamin; Triethylentetramin; 3,3'-Dimethyl-4,4'-diaminodicyclohexylmethan; Methylnonandiamin; Isophorondiamin; 4,4'-Diaminodicyclohexylmethan; Alkanolamine und -diamine handelt.

4. Wässrige Resorcin-Formaldehyd-Latex-Dispersion, enthaltend mindestens einen Haftvermittler aus einem oder mehreren der Ansprüche 1 bis 3.

5. Wässrige Resorcin-Formaldehyd-Latex-Dispersion nach Anspruch 4, **dadurch gekennzeichnet, dass** es sich bei der Resorcin-Formaldehyd-Latex-Dispersion um eine Dispersion der Einzelkomponenten Resorcin und Formaldehyd und/oder Formaldehyd zusammen mit einem Vorkondensat aus Resorcin und Formaldehyd und einer oder mehreren der Latex-Dispersionen, ausgewählt aus der Gruppe: carboxylierte Styrol-Butadien-Copolymere (XSBR-Latex), Nitril-Butadien-Copolymere (NBR-Latex), Polychloropren (CR-Latex), Pyridin-Styrol-Butadien-Copolymere (PSBR-Latex) und/oder Reinacrylat- und/oder Styrol-Acrylat-Copolymere (Acrylat-Latex) und/oder Styrol-Butadien-Vinylpyridin-Copolymer-Latices, handelt.

6. Verfahren zur Herstellung von wässrigen Resorcin-Formaldehyd-Latex-Dispersionen nach Anspruch 4 oder 5, **dadurch gekennzeichnet, dass** mindestens ein Haftvermittler nach einem oder mehreren der Ansprüche 1 bis 3 in die Resorcin-Formaldehyd-Latex-Dispersion eingerührt wird.

7. Verfahren zur Herstellung von wässrigen Resorcin-Formaldehyd-Latex-Dispersionen nach Anspruch 4 oder 5, **dadurch gekennzeichnet, dass** zur Oberflächendesaktivierung mindestens ein Carbodiimid nach den Formeln (I) bis (IV) mit mindestens einem Amin entweder
a) durch Dispergieren mindestens eines pulverförmigen Carbodiimids nach den Formeln (I) bis (IV) in einer Lösung mindestens eines Amins oder
b) durch Zugabe mindestens eines Amins oder einer Lösung mindestens eines Amins zu einer Dispersion mindestens eines der Carbodiimide nach den Formeln (I) bis (IV) hergestellt wird, und anschließend
in die Resorcin-Formaldehyd-Latex-Dispersion eingerührt oder die Resorcin-Formaldehyd-Latex-Dispersion in diese Lösungen aus a) oder b) eingerührt wird.

8. Haftmittelformulierung, enthaltend eine wässrige Resorcin-Formaldehyd-Latex-Dispersion nach Anspruch 4 oder 5 und zusätzlich mindestens ein Aktivierungsmittel.

9. Haftmittelformulierung nach Anspruch 8, **dadurch gekennzeichnet, dass** das Aktivierungsmittel mindestens ein Epoxid ist.

10. Verfahren zur Verbesserung der Haftung von Verstärkungsfasern mit vernetztem Kautschuk und/oder Elastomeren, **dadurch gekennzeichnet, dass**
- die Fasern in eine Haftmittelformulierung nach Anspruch 8 oder 9 eingebracht und anschließend getrocknet werden oder
- die Fasern in ein oder mehreren Schritten mit einem und/oder mehreren der Bestandteil(en) der Haftmittelformulierung nach Anspruch 8 oder 9 behandelt werden.

11. Verfahren zur Verbesserung der Haftung von Verstärkungsfasern mit vernetzten Kautschuken oder Elastomeren, **dadurch gekennzeichnet, dass** die voraktivierten Fasern in eine wässrige Resorcin-Formaldehyd-Latex-Dispersion nach Anspruch 4 oder 5 eingebracht und anschließend getrocknet werden.

12. Haftungsverbesserte Fasern, erhältlich durch Inkontaktbringen der mit Aktivierungsmittel vorbehandelten Fasern mit mindestens einer wässrigen Resorcin-Formaldehyd-Latex-Dispersion nach Anspruch 4 oder 5 oder durch Inkontaktbringen einer nicht-vorbehandelten Faser mit mindestens einer Haftmittelformulierung nach Anspruch 8 oder 9 und anschließende Trocknung (Fixierung) bei Temperaturen von 180 bis 260 °C.

13. Verwendung der wässrigen Resorcin-Formaldehyd-Latex-Dispersionen nach Anspruch 4 oder 5 gegebenenfalls in Anwesenheit von Aktivierungsmitteln zur Verbesserung der Haftfestigkeit zwischen Verstärkungsfasern und Elastomeren in Reifen, Antriebsriemen, Transportbändern und/oder Schläuchen.

14. Verwendung eines Haftvermittlers nach einem oder mehreren der Ansprüche 1 bis 3 gegebenenfalls in Anwesenheit von Aktivierungsmitteln und in einer wässrigen Resorcin-Formaldehyd-Latex-Dispersion zur Verbesserung der Haftfestigkeit zwischen Verstärkungsfasern und Elastomeren in Reifen, Antriebsriemen, Transportbändern und/oder Schläuchen.

## Claims

1. Coupling agent comprising at least one carbodiimide based on compounds of the formula (I)
R'-(-N=C=N-R-)ₘ-R" (I),
in which
m is an integer from 1 to 500,
R = C₁-C₁₈-alkylene, C₅-C₁₈-cycloalkylene, arylene and/or C₇-C₁₈-aralkylene,
R' = R-NCO, R-NHCONHR¹, R-NHCONR¹R² or R-NHCOOR³ and
R"= -NCO, -NHCONHR¹, -NHCONR¹R² or -NHCOO_{R}³,
where R¹ and R² in R' are mutually independently identical or different and are a C₁-C₆-alkyl moiety, C₆-C₁₀-cycloalkyl moiety or C₇-C₁₈-aralkyl moiety, and R³ is as defined for R¹ or is a polyester moiety or a polyamide moiety or -(CH₂)₁-(O-(CH₂)ₖ-O)_{g}-R⁴, -C₆H₄(OH) or -C₆H₃(OH)-((CH₂)ₕ-C₆H₄(OH))_{y}
where l = from 1 to 3, k = from 1 to 3, g = from 0 to 12 , h = from 1 to 2 and y = from 1 to 50,
and
R⁴ = H or C₁-C₄-alkyl,
where these have been surface-deactivated via reaction with at least one amine.

2. Coupling agent according to Claim 1, **characterized in that** the carbodiimide involves compounds of the formulae II, III and IV 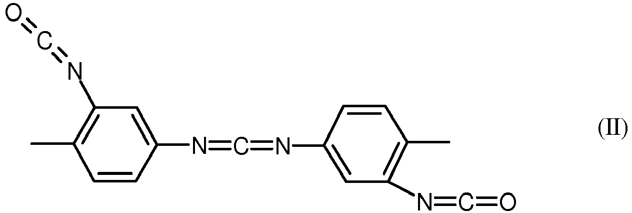 and/or 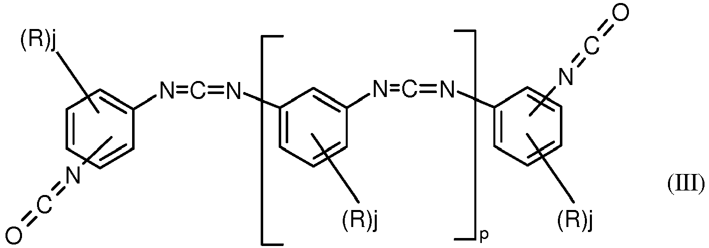 where R = C₁-C₁₈-alkylene, C₅-C₁₈-cycloalkylene, arylene and/or C₇-C₁₈-aralkylene and
j is identical or different within the molecule and is from 1 to 5, and p = from 0 to 500,
and/or 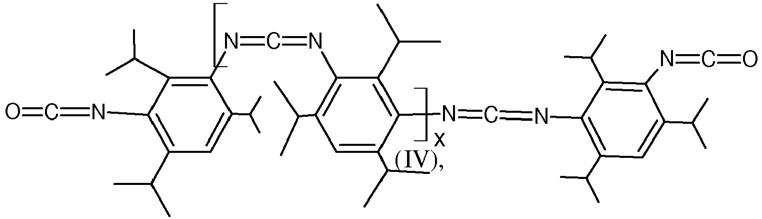 where x = from 1 to 500,
where these have been surface-deactivated via reaction with at least one amine.

3. Coupling agent according to Claim 1 or 2, **characterized in that** the amines involve 1,6-hexamethylenediamine; di-sec-butylamine; ethylenediamine; 1,3-propylenediamine; diethylenetriamine; triethylenetetramine; 3,3'-dimethyl-4,4'-diaminodicyclohexylmethane; methylnonanediamine; isophoronediamine; 4,4'-diaminodicyclohexylmethane; alkanolamines and -diamines.

4. Aqueous resorcinol-formaldehyde-latex dispersion, comprising at least one coupling agent of one or more of Claims 1 to 3.

5. Aqueous resorcinol-formaldehyde-latex dispersion according to Claim 4, **characterized in that** the resorcinol-formaldehyde-latex dispersion involves a dispersion of the individual components resorcinol and formaldehyde, and/or formaldehyde together with a precondensate made of resorcinol and formaldehyde and of one or more of the latex dispersions selected from the following group: carboxylated styrene-butadiene copolymers (XSBR latex), nitrile-butadiene copolymers (NBR latex), polychloroprene (CR latex), pyridine-styrene-butadiene copolymers (PSBR latex) and/or acrylate-only copolymers and/or styrene-acrylate copolymers (acrylate latex) and/or styrene-butadiene-vinylpyridine copolymer latices.

6. Process for producing aqueous resorcinol-formaldehyde-latex dispersions according to Claim 4 or 5, **characterized in that** at least one coupling agent according to one or more of Claims 1 to 3 is incorporated by stirring into the resorcinol-formaldehyde-latex dispersion.

7. Process for producing aqueous resorcinol-formaldehyde-latex dispersions according to Claim 4 or 5, **characterized in that**, for the surface-deactivation process, at least one carbodiimide according to the formulae (I) to (IV) is deactivated with at least one amine either
a) via dispersion of at least one pulverulent carbodiimide according to the formulae (I) to (IV) in a solution of at least one amine or
b) via addition of at least one amine or one solution of at least one amine to a dispersion of at least one of the carbodiimides according to the formulae (I) to (IV), and then
is incorporated by stirring into the resorcinol-formaldehyde-latex dispersion, or the resorcinol-formaldehyde-latex dispersion is incorporated by stirring into these solutions from a) or b).

8. Adhesive formulation, comprising an aqueous resorcinol-formaldehyde-latex dispersion according to Claim 4 or 5 and also at least one activator.

9. Adhesive formulation according to Claim 8, **characterized in that** the activator is at least one epoxide.

10. Process for improving the adhesion of reinforcement fibres to crosslinked rubber and/or elastomers, **characterized in that**
- the fibres are introduced into an adhesive formulation according to Claim 8 or 9 and are then dried, or
- the fibres are treated in one or more steps with one or more of the constituent(s) of the adhesive formulation according to Claim 8 or 9.

11. Process for improving the adhesion of reinforcement fibres to crosslinked rubbers or elastomers, **characterized in that** the preactivated fibres are introduced into an aqueous resorcinol-formaldehyde-latex dispersion according to Claim 4 or 5 and are then dried.

12. Adhesion-improved fibres, obtainable by bringing the activator-pretreated fibres into contact with at least one aqueous resorcinol-formaldehyde-latex dispersion according to Claim 4 or 5, or by bringing a non-pretreated fibre into contact with at least one adhesive formulation according to Claim 8 or 9, and subsequent drying (setting) at temperatures of from 180 to 260°C.

13. Use of the aqueous resorcinol-formaldehyde-latex dispersions according to Claim 4 or 5 optionally in the presence of activators to improve the bond strength between reinforcement fibres and elastomers in tyres, drive belts, conveyor belts and/or hoses.

14. Use of a coupling agent according to one or more of Claims 1 to 3 optionally in the presence of activators and in an aqueous resorcinol-formaldehyde-latex dispersion to improve the bond strength between reinforcement fibres and elastomers in tyres, drive belts, conveyor belts and/or hoses.

## Revendications

1. Promoteur d'adhésion contenant au moins un carbodiimide à base de composés de formule (I)
R'-(-N=C=N-R-)ₘ-R" (I)
dans laquelle
m correspond à un nombre entier de 1 à 500,
R = alkylène en C₁-C₁₈, cycloalkylène en C₅-C₁₈, arylène et/ou aralkylène en C₇-C₁₈,
R' = R-NCO, R-NHCONHR¹, R-NHCONR¹R² ou R-NHCOOR³, et
R" = -NCO, -NHCONHR¹, -NHCONR¹R² ou -NHCOOR³,
R¹ et R² dans R' étant indépendamment l'un de l'autre identiques ou différents, et représentant un radical alkyle en C₁-C₆, cycloalkyle en C₆-C₁₀ ou aralkyle en C₇-C₁₈, et R³ ayant une des significations de R¹ ou représentant un radical polyester ou polyamide ou -(CH₂)₁-(O-(CH₂)ₖ-O)_{g}-R⁴, -C₆H₄(OH) ou -C₆H₃(OH)-((CH₂)ₕ-C₆H₄(OH))_{y}
avec l = 1 à 3, k = 1 à 3, g = 0 à 12, h = 1 à 2 et y = 1 à 50,
et
R⁴ = H ou alkyle en C₁-C₄,
qui ont été désactivés en surface par réaction avec au moins une amine.

2. Promoteur d'adhésion selon la revendication 1, **caractérisé en ce que** le carbodiimide correspond à des composés des formules II, III et IV 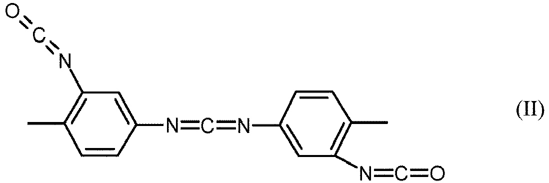 et/ou 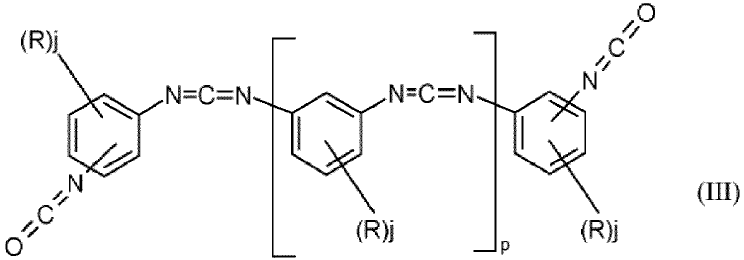 avec R = alkylène en C₁-C₁₈, cycloalkylène en C₅-C₁₈, arylène et/ou aralkylène en C₇-C₁₈, et les j dans la molécule étant identiques ou différents, et signifiant 1 à 5, et p = 0 à 500,
et/ou 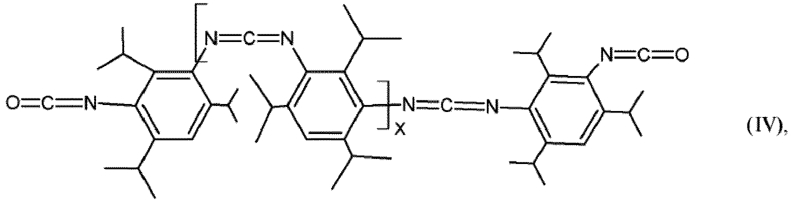 avec x = 1 à 500,
qui ont été désactivés en surface par réaction avec au moins une amine.

3. Promoteur d'adhésion selon la revendication 1 ou 2, **caractérisé en ce que** les amines sont la 1,6-hexaméthylène-diamine ; la di-sec.-butylamine ; l'éthylène-diamine ; la 1,3-propylène-diamine ; la diéthylène-triamine ; la triéthylène-tétramine ; le 3,3'-diméthyl-4,4'-diaminodicyclohexylméthane ; la méthylnonane-diamine ; l'isophorone-diamine ; le 4,4'-diaminodicyclohexylméthane ; des alcanolamines et -diamines.

4. Dispersion aqueuse de résorcine-formaldéhyde-latex, contenant au moins un promoteur d'adhésion selon une ou plusieurs des revendications 1 à 3.

5. Dispersion aqueuse de résorcine-formaldéhyde-latex selon la revendication 4, **caractérisée en ce que** la dispersion de résorcine-formaldéhyde-latex est une dispersion des composants individuels résorcine et formaldéhyde et/ou formaldéhyde conjointement avec un précondensat de résorcine et de formaldéhyde et une ou plusieurs dispersions de latex, choisies dans le groupe constitué par : les copolymères de styrène-butadiène carboxylés (latex XSBR), les copolymères de nitrile-butadiène (latex NBR), le polychloroprène (latex CR), les copolymères de pyridine-styrène-butadiène (latex PSBR) et/ou les copolymères d'acrylate pur et/ou de styrène-acrylate (latex d'acrylate) et/ou les latex de copolymères de styrène-butadiène-vinylpyridine.

6. Procédé de fabrication de dispersions aqueuses de résorcine-formaldéhyde-latex selon la revendication 4 ou 5, **caractérisé en ce qu'**au moins un promoteur d'adhésion selon une ou plusieurs des revendications 1 à 3 est incorporé dans la dispersion de résorcine-formaldéhyde-latex.

7. Procédé de fabrication de dispersions aqueuses de résorcine-formaldéhyde-latex selon la revendication 4 ou 5, **caractérisé en ce que** pour la désactivation de surface, au moins un carbodiimide selon les formules (I) à (IV) est fabriqué avec au moins une amine, soit
a) par dispersion d'au moins un carbodiimide en poudre selon les formules (I) à (IV) dans une solution d'au moins une amine, soit
b) par ajout d'au moins une amine ou d'une solution d'au moins une amine à une dispersion d'au moins un des carbodiimides selon les formules (I) à (IV), puis
incorporé dans la dispersion de résorcine-formaldéhyde-latex ou la dispersion de résorcine-formaldéhyde-latex est incorporée dans ces solutions de a) ou b).

8. Formulation d'agent adhésif, contenant une dispersion aqueuse de résorcine-formaldéhyde-latex selon la revendication 4 ou 5 et en outre au moins un agent d'activation.

9. Formulation d'agent adhésif selon la revendication 8, **caractérisée en ce que** l'agent d'activation est au moins un époxyde.

10. Procédé d'amélioration de l'adhésion de fibres de renforcement avec un caoutchouc réticulé et/ou des élastomères, **caractérisé en ce que**
- les fibres sont introduites dans une formulation d'agent adhésif selon la revendication 8 ou 9, puis séchées, ou
- les fibres sont traitées en une ou plusieurs étapes avec un et/ou plusieurs des constituant(s) de la formulation d'agent adhésif selon la revendication 8 ou 9.

11. Procédé d'amélioration de l'adhésion de fibres de renforcement avec des caoutchoucs réticulés ou des élastomères, **caractérisé en ce que** les fibres préactivées sont introduites dans une dispersion aqueuse de résorcine-formaldéhyde-latex selon la revendication 4 ou 5, puis séchées.

12. Fibres à adhésion améliorée, pouvant être obtenues par mise en contact des fibres prétraitées avec un agent d'activation avec au moins une dispersion aqueuse de résorcine-formaldéhyde-latex selon la revendication 4 ou 5 ou par mise en contact d'une fibre non prétraitée avec au moins une formulation d'agent adhésif selon la revendication 8 ou 9, puis séchage (fixation) à des températures de 180 à 260 °C.

13. Utilisation des dispersions aqueuses de résorcine-formaldéhyde-latex selon la revendication 4 ou 5, éventuellement en présence d'agents d'activation, pour l'amélioration de l'adhésion entre des fibres de renforcement et des élastomères dans des pneus, des courroies d'entraînement, des bandes de transport et/ou des tuyaux.

14. Utilisation d'un promoteur d'adhésion selon une ou plusieurs des revendications 1 à 3, éventuellement en présence d'agents d'activation et dans une dispersion aqueuse de résorcine-formaldéhyde-latex pour l'amélioration de l'adhésion entre des fibres de renforcement et des élastomères dans des pneus, des courroies d'entraînement, des bandes de transport et/ou des tuyaux.
